# EUROPEAN PATENT SPECIFICATION

(11) **EP 3 368 133 B1**
(45) Date of publication and mention of the grant of the patent: **01.04.2020**
(21) Application number: 15907166.1
(22) Date of filing: 30.10.2015
(51) Int. Cl.: A61M 25/10

(54) **DRUG COATED BALLOON CATHETER ARTERIOVENOUS SHUNT**
ARTERIOVENÖSER SHUNT MIT MEDIKAMENTENBESCHICHTETEM BALLONKATHETER
SHUNT ARTÉRIO-VEINEX DE CATHÉTER À BALLONNET ENROBÉ DE MÉDICAMENT

(43) Date of publication of application: 05.09.2018
(73) Proprietor: Acotec Scientific Co. Ltd, Beijing 100176 (CN)
(72) Inventor: SCHAFFNER, Silvio, Beijing 100176 (CN)
(74) Representative: Mitola, Marco
(86) International application number: PCT/IB2015/002090
(87) International publication number: WO 2017/072545

(56) References cited:
- EP-A1- 2 662 109
- EP-A2- 2 532 300
- WO-A1-2015/151877
- CN-A- 103 316 417
- CN-U- 201 814 973
- CN-U- 203 954 434
- US-A1- 2005 177 094
- US-A1- 2014 276 585
- US-A1- 2014 296 832

## Description

The present invention relates to a balloon catheter, as described in the claims, for arteriovenous (AV) shunt.

In particular, AV-shunt patients have high calcified lesions and very high restenosis rate. Therefore, they need dilation of the artery several times with high pressure.

It is known in the art to realize the dilation of a restenosis by means of percutaneous transluminal angioplasty (PTA) balloons.

It is also known in the art to use drug eluted balloon (DEB) catheters or drug coated balloon (DCB) catheters wherein the drug helps the local treatment of the lesion or restenosis, from the inner side of the vessel wall.

Such known catheters have some drawbacks: in fact DEB PTA balloons have small diameters and low inflating (and burst) pressures. In addition, they do not have indication of specific dimensions.

Therefore, known PTA balloons are not able to dilate the restenosis efficiently, in particular for AV-shunt restenosis. Such solutions are known, for example, from US 2014/276585 A1, EP 2662109 A1, EP 2532300 A2, WO 2015/151877 A1.

The purpose of the present invention is that of providing a catheter which overcomes the drawbacks mentioned with reference to the prior art; in other words a catheter for AV shunt which can easily reach the target lesion, completely dilate the restenosis and significantly reduce the restenosis rate.

Such aim is reached by a catheter according to claim 1.

Other embodiments of the catheter according to the invention are described in the subsequent claims.

Further characteristics and advantages of the present invention will be more clearly comprehensible from the description given below of its preferred and non-limiting embodiments, wherein:

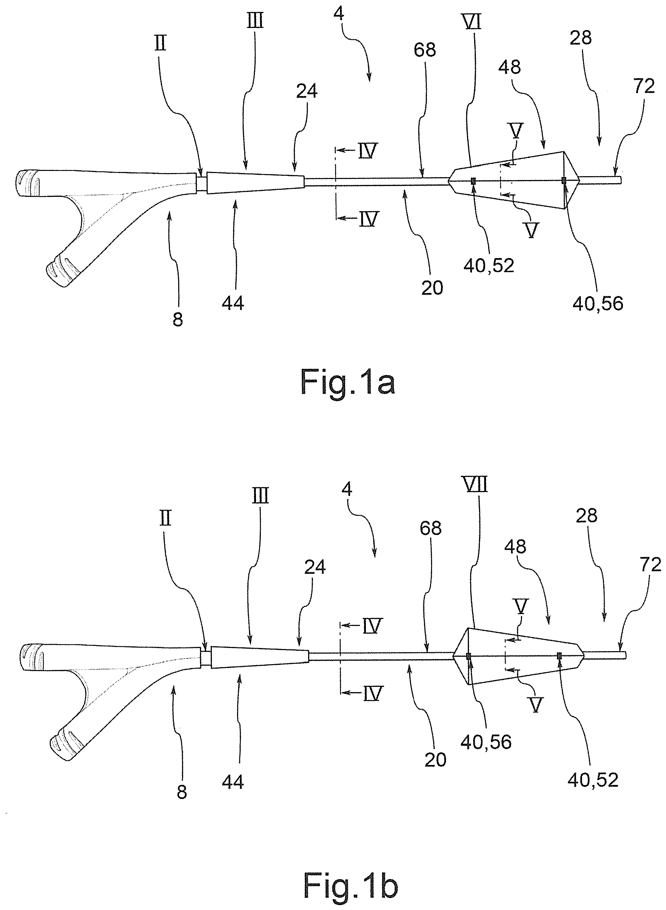

figure 1a shows a perspective view of a drug coated balloon (DCB) catheter according to an embodiment of the present invention;
figure 1b shows a perspective view of a drug coated balloon (DCB) catheter according to another embodiment of the present invention;

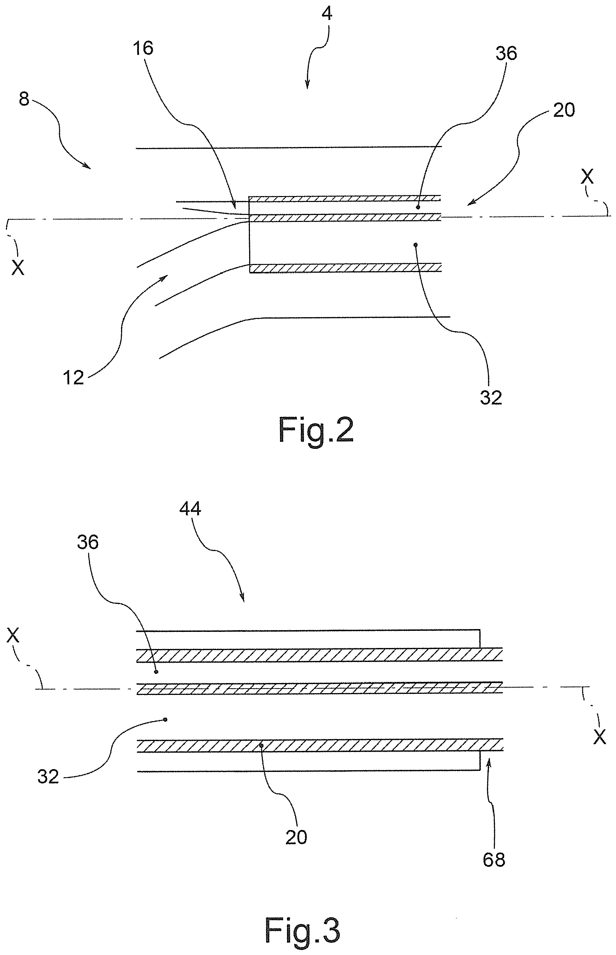

figure 2 shows a longitudinal section view of particular II of the catheter of figures 1a-1b;
figure 3 shows a longitudinal section view of particular III of the catheter of figures 1a-1b;

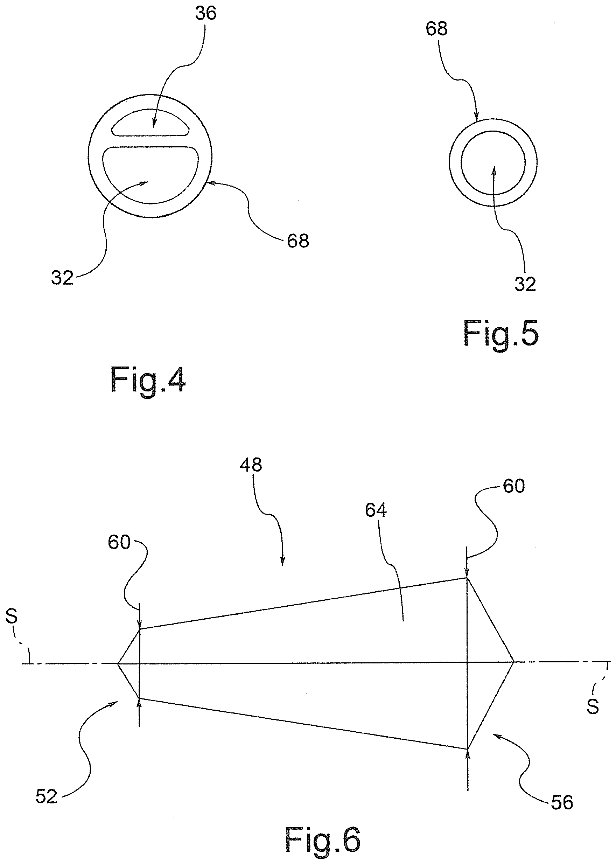

figure 4 figure 4 shows a section view of the catheter of figures 1a, taken along section line IV-IV shown in figures 1a-1b;
figure 5 shows a section view of the catheter of figures 1a-1b, taken along section line V-V shown in figures 1a-1b;
figure 6 is an enlarged view of particular VI of figures 1a-1b.

The elements or parts of elements common to the embodiments described below will be indicated using the same reference numerals.

With reference to the aforementioned figures, reference numeral 4 globally denotes a catheter, in particular, a drug coated balloon (DCB) catheter for releasing drug on a target lesion inside a blood vessel and for dilate a stenosis of a blood vessel, in particular in AV-shunt patients.

The catheter 4 comprises a connector 8 comprising a guide wire channel 12 and an inflation channel 16.

The guide wire channel 12 is useful for housing a guide wire for the guide and the insertion of the catheter 4 in a predetermined vessel.

Moreover, the inflation channel 16 is useful in order to send a pressure fluid to the distal end of the shaft of the catheter. For example the pressure fluid may be gas or a liquid. Moreover, the inflation channel may be used as a perfusion channel, for example, of a contrast liquid.

The catheter 4 comprises a shaft 20 extending from a proximal end 24 to a distal end 28 along an axial direction X-X.

Moreover, the shaft 20 have a guide wire lumen 32 and an inflation lumen 36.

Preferably, said guidewire lumen 32 is between 0.010 inches to 0.037 inches (0.254 to 0.94 mm).

According to a possible embodiment, the catheter has at least a dual-lumen construction, comprising at least two lumens comprising a guidewire lumen 32 and an inflation lumen 36.

According to a possible embodiment, said guidewire lumen 32 and inflation lumen 36 are not coaxial each other. For example said guidewire lumen 32 and inflation lumen 36 are separated by an internal sept 44. For example said internal sept 44 delimits, at least partly, a wall of each of the separated lumens 32,36.

Anyway, the catheter 4 may have further lumens for different purposes.

The shaft 20 is connected to the connector 8 on said proximal end 24.

In particular, the shaft 20 is connected, at its proximal end 24, to the connector 8 so that said guide wire lumen 32 and inflation lumen 36 are mechanically and fluidically connected with said guide wire channel 12 and inflation channel 16 of the connector 8, respectively.

According to an embodiment, the shaft 20 and the connector 8 are made up of a polymer material, such as Polyamide, Pebax, Polycarbonate and similar.

According to the invention, the shaft is provided with an inflatable balloon 48, which is fluidically connected with said inflation lumen 36 in order to be selectively inflated and/or deflated.

According to one embodiment, the balloon 48 is provided to a couple of markers 40, positioned in correspondence of a first end 52 and a second end 56 of the balloon 48, defining the axial length of the balloon itself. In particular, the balloon extends along a prevalent extension S-S.

Advantageously, the balloon 48 is conical and axis-symmetrical around said prevalent extension axis S-S.

The balloon 48 has an increasing diameter 60, moving from the first end 52 to the second end 56, said diameter 60 being measured on a section plane perpendicular to said prevalent extension axis S-S. According to an embodiment said first end 52 faces the proximal end 24 and said second end 56 faces the distal end 28; according to another embodiment the first end 52 faces the distal end 28 and said second end 56 faces the proximal end 24.

Preferably, the diameter 80 of the balloon 48 continuously increases moving from the first end 52 to the second end 56.

According to a possible embodiment, the diameter 80 at the first end 52 is 6 mm.

According to a possible embodiment, the diameter 80 at the second end is 7 mm.

According to further embodiments, the diameter 80 at the second end is 8 mm or 9 mm.

According to an embodiment, the length of the balloon 48 or axial distance from the first end 52 to the second end 56 of the balloon 48 is between 30 mm and 60 mm.

Preferably, said length of the balloon 48 is about 45 mm.

Preferably, said conical balloon 48 is configured so as to have a nominal or working pressure between 12 to 16 bar.

Preferably, said conical balloon 48 is configured so as to have a burst pressure above 25 bar, preferably 30 to 40 bar. According to a possible embodiment the balloon 48 has an increased wall thickness. For example such a thickness is comprised between 0.01 mm and 0.2 mm; preferably such thickness is about double or triple than prior art balloons.

Such an increased wall thickness can be obtained by a single thicker sheath delimiting the balloon surface or by the juxtaposition of two balloons (so called double balloon construction). According to another possible embodiment, the balloon is stiffened by reinforcing fibers.

Advantageously, an external surface 64 of the balloon 48 is coated with a drug for treating restenosis.

Such a drug can be delivered by the balloon 48 on a target lesion or stenosis to be treated and enlarged.

The drug can be of any type, depending on the kind and positioning of lesion to be treated.

According to one embodiment, the drug formulation is a Paclitaxel solution.

Preferably, said drug on the external surface 64 of the balloon 48 has a concentration between 2 to 10 µg/mm².

According to an embodiment of the invention, an external wall 68 of the shaft 20, opposite to said lumens 32,36, is covered with a lubricant.

In this way, said external wall 68 which, during insertion of the catheter into the vessel, contacts the internal wall of the blood vessel or the internal wall of an introducer, sheath, guiding catheter or any other accessory used for intervention, is lubricated and can easily slip into the vessel itself.

According to the invention, the external wall 68 of the shaft 20 is covered with a lubricant except for said inflatable balloon 48.

In this way, the lubricant does not interfere with the action of the drug which has to be delivered on the target lesion.

The distal end 28 of the shaft 20 comprises a flexible tip 72 which aims the catheter to be cannulated inside blood vessels.

According to one embodiment, said flexible tip 72 is covered with lubricant too.

Advantageously, said guidewire lumen 32 is internally covered with a lubricant.

As it can be seen from the description, the catheter according to the invention makes it possible to overcome the drawbacks mentioned with reference to the prior art.

In particular, it is possible to treat efficiently an AV-shunt restenosis by means of a conical balloon which can reach the target lesion easily, and which can dilate the restenosis completely using high pressure so as to get the correct lumen.

Moreover, it is possible to treat the lesion by means of drug which is applied to the inner wall of the vessel to be dilated.

Moreover the specific geometry of the conical balloon implies that the external surface of the balloon is not constant along the axial extension of the balloon: in particular the external surface is smaller towards the portion with smaller diameter and it is bigger towards the portion with bigger diameter.

Therefore, in the catheter according to the present invention, the concentration of the drug on the balloon surface is differentiated along the axial extension of the balloon: in other words the concentration of applied drug is lower towards the portion with smaller diameter and it is higher towards the portion with higher diameter. In this way, the concentration of drug applied to the balloon compensates the asymmetrical geometry of the balloon along its axial extension. Therefore the drug concentration per mm2 balloon surface is constant over the balloon to reach homogeneous drug application to the tissue to be treated.

Moreover, the specific shape and dimension of the conical balloon according to the invention grants both a good dilation of the restenosis, since the balloon acts as a sort of wedge in order to expand the lumen of the blood vessel.

Moreover the use of lubricant helps the catheter reaching the target lesion without jamming within blood vessels and without damaging them during vessel cannulation.

## Claims

1. A drug coated balloon (DCB) catheter (4) comprising
- a connector (8),
- a shaft (20) extending from a proximal end (24) to a distal end (28) along an axial direction (X-X) and having a guidewire lumen (32) and an inflation lumen (36), the shaft being connected to the connector (8) on said proximal end (24),
- the shaft (20) being provided with an inflatable balloon (48), fluidically connected with said inflation lumen (36) in order to be selectively inflated and/or deflated, wherein
- the balloon (48) is conical and axis-symmetrical around a prevalent extension axis (S-S),
- wherein the balloon (48) has an increasing diameter (60), moving from a first end (52) to a second end (56), said diameter (60) being measured on a section plane perpendicular to said prevalent extension axis (S-S),
- wherein an external surface (64) of the balloon (48) is coated with a drug for treating restenosis,
- **characterised in that** the concentration of drug applied to the balloon surface is lower towards the portion with smaller diameter and it is higher towards the portion with higher diameter, so as to get a constant drug concentration over the balloon surface, along its axial extension.

2. Drug coated balloon (DCB) catheter (4) according to claim 1, wherein the diameter (60) of the balloon (48) continuously increases moving from the first end (52) to the second end (56).

3. Drug coated balloon (DCB) catheter (4) according to claim 1 or 2, wherein said first end (52) faces the proximal end (24) and said second end (56) faces the distal end (28).

4. Drug coated balloon (DCB) catheter (4) according to claim 1 or 2, wherein said first end (52) faces the distal end (28) and said second end (56) faces the proximal end (24).

5. Drug coated balloon (DCB) catheter (4) according to any one of previous claims, wherein the diameter (60) at the first end (52) is 6 mm.

6. Drug coated balloon (DCB) catheter (4) according to any one of previous claims, wherein the diameter at the second end (56) is 7 mm.

7. Drug coated balloon (DCB) catheter (4) according to any one of claims 1 to 5, wherein the diameter (60) at the second end (56) is 8 mm.

8. Drug coated balloon (DCB) catheter (4) according to any one of claims 1 or 5, wherein the diameter (60) at the second end (56) is 9 mm.

9. Drug coated balloon (DCB) catheter (4) according to any one of the previous claims, wherein the length of the balloon (48) or axial distance from the first end (52) to the second end (56) of the balloon (48) is between 30 mm and 60 mm.

10. Drug coated balloon (DCB) catheter (4) according to claim 9, wherein said length of the balloon (48) is about 45 mm.

11. Drug coated balloon (DCB) catheter (4) according to any one of the previous claims, wherein said conical balloon (48) is configured so as to have a nominal or working pressure between 12 to 16 bar.

12. Drug coated balloon (DCB) catheter (4) according to any one of the previous claims, wherein said conical balloon (48) is configured so as to have a burst pressure above 25 bar, preferably, from 30 to 40 bar.

13. Drug coated balloon (DCB) catheter (4) according to any one of the previous claims, wherein said drug is a Paclitaxel solution.

14. Drug coated balloon (DCB) catheter (4) according to any one of the previous claims, wherein said drug on the external surface of the balloon has a concentration between 2 to 10 µg/mm².

15. Drug coated balloon (DCB) catheter (4) according to any one of the previous claims, wherein said guidewire lumen (32) is between 0.010 inches to 0.037 inches (0.254 to 0.94 mm).

16. Drug coated balloon (DCB) catheter (4) according to any one of the previous claims, wherein the catheter (4) has at least a dual-lumen construction provided with at least two lumens comprising said guidewire lumen (32) and said inflation lumen (36), wherein said lumens (32,36) are separated each other by an internal sept (44).

17. Drug coated balloon (DCB) catheter (4) according to any one of the previous claims, wherein an external wall (68) of the shaft (20), opposite to said lumens (32,36), is covered with a lubricant.

18. Drug coated balloon (DCB) catheter (4) according to claim 17, wherein the entire external wall (68) of the shaft (20) is covered with a lubricant except for said balloon (48).

19. Drug coated balloon (DCB) catheter (4) according to any one of the previous claims, wherein the balloon (48) has an increased wall thickness, comprised between 0.01 mm and 0.2 mm.

20. Drug coated balloon (DCB) catheter (4) according to claim 19, wherein such an increased wall thickness is obtained by a single sheath delimiting the balloon surface.

21. Drug coated balloon (DCB) catheter (4) according to claim 19, wherein such an increased wall thickness is obtained by the juxtaposition of two balloons (48).

22. Drug coated balloon (DCB) catheter (4) according to any one of the previous claims, wherein the balloon (48) is stiffened by reinforcing fibers.

## Patentansprüche

1. Medikamentenbeschichteter Ballonkatheter (DCB) (4), umfassend
- einen Verbinder (8),
- einen Schaft (20), der sich von einem nahen Ende (24) zu einem fernen Ende (28) entlang einer axialen Richtung (X-X) erstreckt und ein Leitdrahtlumen (32) und ein Aufblaslumen (36) aufweist, wobei der Schaft mit dem Verbinder (8) an dem nahen Ende (24) verbunden ist,
- wobei der Schaft (20) mit einem aufblasbaren Ballon (48) versehen ist, der fluidleitend mit dem Aufblaslumen (36) verbunden ist, um selektiv aufgeblasen und/oder abgelassen zu werden,
wobei
- der Ballon (48) konisch und achsensymmetrisch um eine vorherrschende Verlängerungsachse (S-S) ist,
- wobei der Ballon (48) einen zunehmenden Durchmesser (60) aufweist, sich von einem ersten Ende (52) zu einem zweiten Ende (56) bewegend, wobei der Durchmesser (60) auf einer Schnittebene senkrecht zu der vorherrschenden Verlängerungsachse (S-S) gemessen wird,
- wobei eine äußere Oberfläche (64) des Ballons (48) mit einem Medikament zum Behandeln von Restenose beschichtet ist,
- **dadurch gekennzeichnet, dass** die Konzentration von auf der Ballonoberfläche aufgebrachtem Medikament in Richtung des Abschnitts mit kleinerem Durchmesser geringer ist und sie in Richtung des Abschnitts mit größerem Durchmesser höher ist, um eine gleichbleibende Medikamentenkonzentration über die Ballonoberfläche entlang ihrer axialen Ausdehnung zu erhalten.

2. Medikamentenbeschichteter Ballonkatheter (DCB) (4) nach Anspruch 1, wobei der Durchmesser (60) des Ballons (48) sich von dem ersten Ende (52) zu dem zweiten Ende (56) bewegend kontinuierlich vergrößert.

3. Medikamentenbeschichteter Ballonkatheter (DCB) (4) nach Anspruch 1 oder 2, wobei das erste Ende (52) dem nahen Ende (24) gegenüberliegt und das zweite Ende (56) dem fernen Ende (28) gegenüberliegt.

4. Medikamentenbeschichteter Ballonkatheter (DCB) (4) nach Anspruch 1 oder 2, wobei das erste Ende (52) dem fernen Ende (28) gegenüberliegt und das zweite Ende (56) dem nahen Ende (24) gegenüberliegt.

5. Medikamentenbeschichteter Ballonkatheter (DCB) (4) nach einem der vorhergehenden Ansprüche, wobei der Durchmesser (60) an dem ersten Ende (52) 6 mm beträgt.

6. Medikamentenbeschichteter Ballonkatheter (DCB) (4) nach einem der vorhergehenden Ansprüche, wobei der Durchmesser (60) an dem zweiten Ende (56) 7 mm beträgt.

7. Medikamentenbeschichteter Ballonkatheter (DCB) (4) nach einem der Ansprüche 1 bis 5, wobei der Durchmesser (60) an dem zweiten Ende (56) 8 mm beträgt.

8. Medikamentenbeschichteter Ballonkatheter (DCB) (4) nach einem der Ansprüche 1 bis 5, wobei der Durchmesser (60) an dem zweiten Ende (56) 9 mm beträgt.

9. Medikamentenbeschichteter Ballonkatheter (DCB) (4) nach einem der vorhergehenden Ansprüche, wobei die Länge des Ballons (48) oder axiale Entfernung von dem ersten Ende (52) zu dem zweiten Ende (56) des Ballons (48) zwischen 30 mm und 60 mm beträgt.

10. Medikamentenbeschichteter Ballonkatheter (DCB) (4) nach Anspruch 9, wobei die Länge des Ballons (48) etwa 45 mm beträgt.

11. Medikamentenbeschichteter Ballonkatheter (DCB) (4) nach einem der vorhergehenden Ansprüche, wobei der konische Ballon (48) so ausgelegt ist, einen nominellen oder Arbeitsdruck zwischen 12 und 16 bar aufzuweisen.

12. Medikamentenbeschichteter Ballonkatheter (DCB) (4) nach einem der vorhergehenden Ansprüche, wobei der konische Ballon (48) so ausgelegt ist, einen Berstdruck von über 25 bar aufzuweisen, bevorzugt von 30 bis 40 bar.

13. Medikamentenbeschichteter Ballonkatheter (DCB) (4) nach einem der vorhergehenden Ansprüche, wobei das Medikament eine Paclitaxellösung ist.

14. Medikamentenbeschichteter Ballonkatheter (DCB) (4) nach einem der vorhergehenden Ansprüche, wobei das Medikament auf der äußeren Oberfläche des Ballons eine Konzentration zwischen 2 bis 10 µg/mm² aufweist.

15. Medikamentenbeschichteter Ballonkatheter (DCB) (4) nach einem der vorhergehenden Ansprüche, wobei das Leitdrahtlumen (32) zwischen 0,010 Inch bis 0,037 Inch (0,254 bis 0,94 mm) ist.

16. Medikamentenbeschichteter Ballonkatheter (DCB) (4) nach einem der vorhergehenden Ansprüche, wobei der Katheter (4) mindestens einen Duallumenaufbau aufweist, ausgestattet mit mindestens zwei Lumen, umfassend das Leitdrahtlumen (32) und das Aufblaslumen (36), wobei die Lumen (32, 36) voneinander durch ein inneres Septum (44) getrennt sind.

17. Medikamentenbeschichteter Ballonkatheter (DCB) (4) nach einem der vorhergehenden Ansprüche, wobei eine äußere Wand (68) des Schafts (20), gegenüber den Lumen (32, 36), mit einem Gleitmittel bedeckt ist.

18. Medikamentenbeschichteter Ballonkatheter (DCB) (4) nach Anspruch 17, wobei die gesamte äußere Wand (68) des Schafts (20), mit Ausnahme des Ballons (48), mit einem Gleitmittel bedeckt ist.

19. Medikamentenbeschichteter Ballonkatheter (DCB) (4) nach einem der vorhergehenden Ansprüche, wobei der Ballon (48) eine vergrößerte Wanddicke aufweist, umfasst zwischen 0,01 mm und 0,2 mm.

20. Medikamentenbeschichteter Ballonkatheter (DCB) (4) nach Anspruch 19, wobei eine solche vergrößerte Wanddicke durch eine einzige, die Ballonoberfläche abgrenzende Umhüllung erhalten ist.

21. Medikamentenbeschichteter Ballonkatheter (DCB) (4) nach Anspruch 19, wobei eine solche vergrößerte Wanddicke durch die Nebeneinanderstellung von zwei Ballons (48) erhalten ist.

22. Medikamentenbeschichteter Ballonkatheter (DCB) (4) nach einem der vorhergehenden Ansprüche, wobei der Ballon (48) durch Verstärkungsfasern versteift ist.

## Revendications

1. Cathéter à ballonnet enrobé de médicament (DCB) (4) comprenant
- un connecteur (8),
- une tige (20) s'étendant d'une extrémité proximale (24) à une extrémité distale (28) le long d'une direction axiale (X-X) et comportant une lumière de fil-guide (32) et une lumière de gonflage (36), la tige étant raccordée au connecteur (8) sur ladite extrémité proximale (24),
- la tige (20) étant pourvue d'un ballonnet gonflable (48), en raccordement fluidique avec ladite lumière de gonflage (36) afin d'être sélectivement gonflé et/ou dégonflé,
dans lequel
- le ballonnet (48) est conique et présente une symétrie axiale autour d'un axe d'extension principal (S-S),
- dans lequel le ballonnet (48) a un diamètre croissant (60), en allant d'une première extrémité (52) à une deuxième extrémité (56), ledit diamètre (60) étant mesuré sur un plan de section perpendiculaire audit axe d'extension principal (S-S),
- dans lequel une surface externe (64) du ballonnet (48) est enrobée d'un médicament pour traiter une resténose,
- **caractérisé en ce que**
la concentration de médicament appliqué sur la surface du ballonnet est moins élevée en direction de la partie ayant un plus petit diamètre et est plus élevée en direction de la partie ayant un plus grand diamètre, de manière à avoir une concentration constante de médicament sur la surface du ballonnet, le long de son extension axiale.

2. Cathéter à ballonnet enrobé de médicament (DCB) (4) selon la revendication 1, dans lequel le diamètre (60) du ballonnet (48) augmente en continu en allant de la première extrémité (52) à la seconde extrémité (56).

3. Cathéter à ballonnet enrobé de médicament (DCB) (4) selon la revendication 1 ou 2, dans lequel ladite première extrémité (52) fait face à l'extrémité proximale (24) et ladite seconde extrémité (56) fait face à l'extrémité distale (28).

4. Cathéter à ballonnet enrobé de médicament (DCB) (4) selon la revendication 1 ou 2, dans lequel ladite première extrémité (52) fait face à l'extrémité distale (28) et ladite seconde extrémité (56) fait face à l'extrémité proximale (24).

5. Cathéter à ballonnet enrobé de médicament (DCB) (4) selon l'une quelconque des revendications précédentes, dans lequel le diamètre (60) au niveau de la première extrémité (52) est de 6 mm.

6. Cathéter à ballonnet enrobé de médicament (DCB) (4) selon l'une quelconque des revendications précédentes, dans lequel le diamètre au niveau de la seconde extrémité (56) est de 7 mm.

7. Cathéter à ballonnet enrobé de médicament (DCB) (4) selon l'une quelconque des revendications 1 à 5, dans lequel le diamètre (60) au niveau de la seconde extrémité (56) est de 8 mm.

8. Cathéter à ballonnet enrobé de médicament (DCB) (4) selon l'une quelconque des revendications 1 ou 5, dans lequel le diamètre (60) au niveau de la seconde extrémité (56) est de 9 mm.

9. Cathéter à ballonnet enrobé de médicament (DCB) (4) selon l'une quelconque des revendications précédentes, dans lequel la longueur du ballonnet (48) ou la distance axiale de la première extrémité (52) à la seconde extrémité (56) du ballonnet (48) est entre 30 mm et 60 mm.

10. Cathéter à ballonnet enrobé de médicament (DCB) (4) selon la revendication 9, dans lequel ladite longueur du ballonnet (48) est d'environ 45 mm.

11. Cathéter à ballonnet enrobé de médicament (DCB) (4) selon l'une quelconque des revendications précédentes, dans lequel ledit ballonnet conique (48) est configuré de manière à avoir une pression nominale ou de travail entre 12 et 16 bars.

12. Cathéter à ballonnet enrobé de médicament (DCB) (4) selon l'une quelconque des revendications précédentes, dans lequel ledit ballonnet conique (48) est configuré pour avoir une pression de rupture supérieure à 25 bars, de préférence, de 30 à 40 bars.

13. Cathéter à ballonnet enrobé de médicament (DCB) (4) selon l'une quelconque des revendications précédentes, dans lequel ledit médicament est une solution de paclitaxel.

14. Cathéter à ballonnet enrobé de médicament (DCB) (4) selon l'une quelconque des revendications précédentes, dans lequel ledit médicament sur la surface externe du ballonnet a une concentration comprise entre 2 et 10 µg/mm².

15. Cathéter à ballonnet enrobé de médicament (DCB) (4) selon l'une quelconque des revendications précédentes, dans lequel ladite lumière de fil-guide (32) est comprise entre 0,010 pouce et 0,037 pouce (0,254 à 0,94 mm).

16. Cathéter à ballonnet enrobé de médicament (DCB) (4) selon l'une quelconque des revendications précédentes, dans lequel le cathéter (4) a au moins une construction à double lumière dotée d'au moins deux lumières comprenant ladite lumière de fil-guide (32) et ladite lumière de gonflage (36), dans lequel lesdites lumières (32, 36) sont séparées l'une de l'autre par un septum interne (44).

17. Cathéter à ballonnet enrobé de médicament (DCB) (4) selon l'une quelconque des revendications précédentes, dans lequel une paroi externe (68) de la tige (20), opposée auxdites lumières (32, 36), est recouverte d'un lubrifiant.

18. Cathéter à ballonnet enrobé de médicament (DCB) (4) selon la revendication 17, dans lequel la totalité de la paroi externe (68) de la tige (20) est recouverte d'un lubrifiant à l'exception dudit ballonnet (48).

19. Cathéter à ballonnet enrobé de médicament (DCB) (4) selon l'une quelconque des revendications précédentes, dans lequel le ballonnet (48) a une épaisseur de paroi augmentée, comprise entre 0,01 mm et 0,2 mm.

20. Cathéter à ballonnet enrobé de médicament (DCB) (4) selon la revendication 19, dans lequel une telle épaisseur de paroi augmentée est obtenue par une gaine unique délimitant la surface du ballonnet.

21. Cathéter à ballonnet enrobé de médicament (DCB) (4) selon la revendication 19, dans lequel une telle épaisseur de paroi augmentée est obtenue par la juxtaposition de deux ballonnets (48).

22. Cathéter à ballonnet enrobé de médicament (DCB) (4) selon l'une quelconque des revendications précédentes, dans lequel le ballonnet (48) est rigidifié par des fibres de renforcement.
